# EUROPEAN PATENT APPLICATION

(11) **EP 2 500 050 A1**
(43) Date of publication of application: **19.09.2012**
(21) Application number: 11158408.2
(22) Date of filing: 16.03.2011
(51) Int. Cl.: A61M 5/145

(54) **Drive head for a syringe pump and method for operating a drive head of a syringe pump**

(71) Applicant: Fresenius Kabi Deutschland GmbH, 61352 Bad Homburg v.d.H. (DE)
(72) Inventor: Rochette, Francois, 38140, Apprieu (FR)
(74) Representative: Richly, Erik

(57) **Abstract**

The invention relates to a drive head for a syringe pump, comprising:
- a contact portion (3) configured to bear against a plunger (501) of a syringe (500) in order to move the plunger (501) relative to a barrel (503) of the syringe, wherein the plunger (501) comprises a main portion (5011) and a flange (502) protruding from the main portion;
- at least one holding element (41, 42) for holding the flange (502) of the syringe plunger (501) in order to prevent a siphon movement of the plunger (501) away from the contact portion (3);
- means (8) for generating a rotational and/or a linear movement of the holding element (41, 42) in order to move the holding element (41, 42) from a first position into a second position and vice versa, wherein in the first position the holding element (41, 42) holds the flange (502) of the syringe plunger (501) while in the second position the holding element (41, 42) is positioned with a distance to the flange (502).

According to the invention the means (8) are configured in such a way that after arranging a syringe at the drive head such that the plunger (501) bears against the contact portion (3) the holding element (41, 42) will bear against a peripheral edge (5021) of the plunger flange (502) when being moved into the first position.

The invention also relates to a method for operating a drive head of a syringe pump.

## Description

The invention relates to a drive head for a syringe pump according to the preamble of claim 1 and to a method for operating a syringe pump according to the preamble of claim 11.

Syringe pumps for emptying a syringe are known from the prior art. For example, syringe pumps are used in the medical field in order to infuse a liquid contained in a syringe into a human body. The known syringe pumps typically comprise a drive head connected to a drive mechanism configured to generate a linear movement of the drive head such that a plunger of the syringe will be moved within the syringe barrel by the drive head. It is also known to arrange a pressure sensor at the drive head in order to be able to measure an inner pressure of the syringe during operation of the syringe pump. A syringe pump including such a drive head is disclosed, for example, in EP 0 916 353 A1.

It is an object of the invention to provide a drive head for a syringe pump that prevents an anti-siphon movement of a syringe plunger as effective as possible. It further is an object of the invention to provide a corresponding method for operating a syringe pump drive head.

These problems are solved by the drive head according to claim 1 and the method according to claim 11. Embodiments of the invention are defined in the dependent claims.

According to the invention a drive head for a syringe pump is provided, comprising
- a contact portion configured to bear against a plunger of a syringe in order to move the plunger relative to a barrel of the syringe, the plunger comprising a main portion and a flange protruding from the main portion; and
- at least one holding element for holding the flange of the syringe plunger in order to prevent a siphon movement of the plunger away from the contact portion,
- means for generating a rotational and/or a linear movement of the holding element in order to move the holding element from a first position into a second position and vice versa, wherein in the first position the holding element holds the flange of the syringe plunger while in the second position the holding element is positioned with a distance to the flange, wherein
- the means are configured in such a way that after arranging a syringe at the drive head such that the plunger bears against the contact portion the holding element will bear against a peripheral edge of the plunger flange when being moved into the first position.

The "first position" of the holding element thus corresponds to a closed position in which it holds the plunger flange, while the "second position" of the holding element corresponds to an open position used for arranging the syringe at the drive head. In particular, the means generating the movement of the holding element produce both a rotational and a linear movement, wherein the holding element is rotated along an axis extending parallel to the direction of movement of the drive head and moved axially along an axis extending parallel to the direction of movement of the drive head.

The means for generating the movement of the holding element may further be configured in such a way that in the second position the holding element is positioned with a greater distance from the contact portion than in the first position, i.e. the first position of the holding element corresponds to a closed and backward position, whereas the second position of the holding element corresponds to an opened and forward position. It is, however, also possible that the means for generating a movement of the holding device generate a rotational movement only.

For example, moving the holding element towards the second position comprises rotating the holding element away from a position where the syringe plunger is supposed to be arranged, while simultaneously or successively moving the holding element linearly into the forward position. Correspondingly, moving the holding element towards the first position comprises rotating the holding element towards the syringe plunger, while simultaneously or successively moving the holding element linearly into the backward position.

According to an embodiment of the invention, the means for generating the movement of the holding element comprise resilient means for pre-tensioning the holding element such the holding element tends to return into the first position. For example, at least one spring is used to pre-tension the holding element such that the holding element has to be moved from the first into the second position against the return force of the spring. Accordingly, the holding element can move back automatically into the first position under the impact of the return force of the spring. In particular, the resilient means pre-tension the holding element both with respect to the rotational movement (in order to make sure that syringes with different plunger flange diameters will be safely held) and the linear movement (in order to adapt to different flange thicknesses).

Further more, in the second position the holding element may be positioned with a distance from the contact portion that is smaller than the thickness of the plunger flange such that the holding element will not pass the peripheral edge of the plunger flange when moving from the second into the first position. It is noted that the "flange" of the syringe plunger is a (e.g. disc-shaped) section of the plunger that has larger diameter than the main portion of the plunger, wherein the flange may be arranged at an end of the plunger main portion. The syringe is arranged at the drive head in such a way that a backside of the plunger flange bears against the contact portion of the drive head.

In particular, the means for generating the movement of the holding element limit the axial movement of the holding element in such a way that in the second position (the open and forward position) the distance (along the direction of the axial movement of the holding element) between the holding element and the contact portion of the drive head in its retracted/activated position is not larger than 1 mm or 0.5 mm. It is also possible that the distance between the holding element and the contact portion of the drive head is zero. Thus, it is ensured that the holding element always bears against the peripheral edge of the flange instead of bearing against the main portion of the plunger. This may provide a more reproducible holding of the syringe plunger as the plunger flange of a syringe typically has a disc like shape, whereas the cross section of the plunger main portion may vary for different syringes. Furthermore, an inner surface of the holding element that faces towards the syringe and that bears against the plunger flange in the first position of the holding element may have a length (measured along the direction of the axial movement of the holding element) of at least 2.5 mm to make sure that syringe plungers with a different thickness of their flanges are captured.

According to another embodiment of the invention, the holding element comprises a gripping element having a first section that in the first position of the holding element bears against the peripheral edge of the plunger flange and a second section that in the first position of the holding element is located with a distance from the main portion of the plunger while engaging the flange on a side that faces away from the contact portion.

The holding element thus is designed in such a manner that it holds the plunger via the interaction of its first section, i.e. by pressing against the peripheral edge of the plunger flange.

The "peripheral edge" of the flange is a circumferential surface (surrounding the longitudinal axis of the plunger) arranged between a front surface (that is orientated perpendicular to the longitudinal axis of the plunger and faces towards the syringe barrel) and a back surface (orientated perpendicular to the longitudinal axis of the plunger and facing away from the syringe barrel) of the plunger.

Further, the plunger flange may additionally be held by the second section of the gripping element, i.e. the means for generating the movement of the holding element are configured in such a way that when moving the holding element from the second (open) position into the first (closed) position the holding element is moved backwards towards the contact portion of the drive head until it bears against the front surface of the syringe plunger. It is noted, however, that it is also possible that in the closed position of the holding element the second section of the gripping element does not bear against the plunger flange. Rather, the second section may neither bear against the main plunger portion nor against the flange, i.e. the second section of the gripping element is positioned with a distance from the flange, wherein, however, it still "engages" the flange as a security measure in case that the frictional force between the first section of the gripping element and the peripheral edge of the flange is not high enough to prevent a siphon movement of the syringe plunger, i.e. a movement evoked by a low pressure in the syringe.

According to another example of the invention, the second section of the gripping element protrudes beyond the first section such that a step is formed between the first and the second section. The "height" of the step is adapted to the height of the flange, i.e. the difference between the diameter of the flange section and the main section of the plunger. The step is smaller than the height of the flange in order to assure that the first portion will not be brought into contact with the main portion of the plunger when the holding element is moved into its closed position in which the first section bears against the peripheral flange edge. For example, the second section of the gripping element protrudes at least 1 mm or at least 1.5 mm beyond the first section. In another example, the second section of the gripping element protrudes at most 1.3 mm, 1.5 mm or 2 mm beyond the first section.

It is possible that the step between the first and the second section extends over essentially the whole length of the gripping element. In particular, the gripping element has a longitudinally extending shape, i.e. it has a length along a first direction that is larger (e.g. at least three times larger) than its dimensions perpendicular to the longitudinal extension. In addition, the gripping element can be curved to grip the flange over a greater distance.

Further, the second section may comprise a tapered portion that faces towards the syringe when the drive head is in operation, i.e. when the gripping element is in the closed position. In other words, the second section of the gripping element is tapered towards the syringe (the syringe plunger) in order to keep the possibility of an interaction between the second section of the gripping element and the plunger as low as possible.

According to another embodiment of the invention, the holding element comprises a rotatably mounted shaft connected to the gripping element so as to rotate the gripping element from the open (second) position in which it does not bear against the syringe flange into the closed (first) position in which it bears against the syringe flange, and vice versa.

Further, the drive head may comprise a first and a second holding element that each can be moved from a first position into a second position by the means, wherein in the first position both the first and the second holding element bear against the peripheral edge of the plunger flange such that the syringe plunger is centered between the first and the second holding element.

In particular, the drive head comprises a first and a second holding element that are rotatably coupled such that a rotation of the first holding element generates a rotation of the second holding element.

It is noted, however, that providing two rotatable holding element is only optional. It is also possible that the drive head comprises only a single rotatable holding element and, for example, a non-rotatable element, wherein the syringe plunger is centered between the rotatable holding element and the non-rotatable element (which at least is not rotatable relative to the drive head housing).

According to another variant of the invention, the drive head comprises a housing wherein an opening is arranged in the housing and the shaft extends through the opening. For example, the shaft is mounted and configured in such a way that it extends through the opening with a distance from an inner edge of the opening, i.e. the holding element is floatingly mounted. Instead, the shaft can be mounted via a support arranged within the drive head housing, e.g. via a pressure sensor support carrying a pressure sensor. Thus, a movement of the holding element is not transferred to the housing such that a perturbation of a pressure sensor that may be connected to the housing is avoided as much as possible.

A floatingly mounted holding element, however, is described in another European patent application entitled "Drive head for a syringe pump" filed by the same applicant and on the same day as the present application, this other European patent application being hereby incorporated by reference.

The first and the second section of the gripping element may be integrally formed, i.e. the first and the second section are formed of a single material piece. For example, the gripping element and/or the whole holding element is formed as a single piece part.

The invention also relates to a syringe pump comprising a drive head as described above.

Further, the invention is related to method for operating a drive head of a syringe pump, comprising the steps of:
- providing a drive head for a syringe pump;
- arranging a syringe on the drive head, the syringe comprising a plunger having a main portion and a flange protruding from the main portion, wherein the drive head comprises a contact portion configured to bear against the plunger of the syringe in order to move the plunger relative to a barrel of the syringe, and at least one holding element for holding the flange of the syringe plunger in order to prevent a siphon movement of the plunger away from the contact portion; and
- moving the holding element into a (closed) position in which the holding element bears against the peripheral edge of the plunger flange.

For example, the holding element is moved from a first position (closed position) into a second position (open position) prior to the arrangement of the syringe at the drive head and is returned into the first position after having arranged the syringe, wherein in the second position the holding element is positioned with a distance from the flange, whereas in the first position the holding element bears against the peripheral edge of the plunger flange.

As set forth above, the holding element may be moved from the first into the second position against the return force of resilient means and may be returned into the first position by means of the return force of the resilient means.

In particular, the drive head provided is a drive head as described above. For example, the gripping element has a first and a second section, the second section protruding beyond the first section, wherein the holding element is moved into the closed position in which the first section bears against the peripheral edge of the plunger flange, while the second section is located in a distance to main portion of the plunger. That is, the second portion does not bear against the main portion of the syringe plunger. It is, however, possible that the second section bears against an inner surface (front surface) of the plunger flange when the holding element is in its closed position, the inner flange surface facing away from the contact portion of the drive head (when the syringe is arranged at the drive head). The second section may also engage the inner surface of the flange without bearing against it.

Also, the holding element can be moved into the closed position in such a way that the first section bears against the peripheral edge of the plunger flange while the second section is located in a distance to the plunger, i.e. it neither bears against the main section of the plunger nor against the plunger flange. The syringe plunger thus is held in position exclusively via the first section of the gripping element.

The embodiments of the invention will be described with more detail hereinafter with reference to the drawings, in which
- Figure 1: illustrates a sectional view of a syringe pump drive head according to an embodiment of the invention;
- Figure 2: illustrates a sectional view of the drive head according to Figure 1 from another point of view;
- Figure 3: illustrates an exploded view of components of the drive head of Figure 1;
- Figure 4: is another illustration of the components of Figure 3;
- Figure 5: illustrates another exploded view of the drive head of Figure 1;
- Figure 6: is a perspective view of the interior of the drive head according to Fig-ures 1 to 5; and
- Figures 7 - 11: show further views of the drive head.

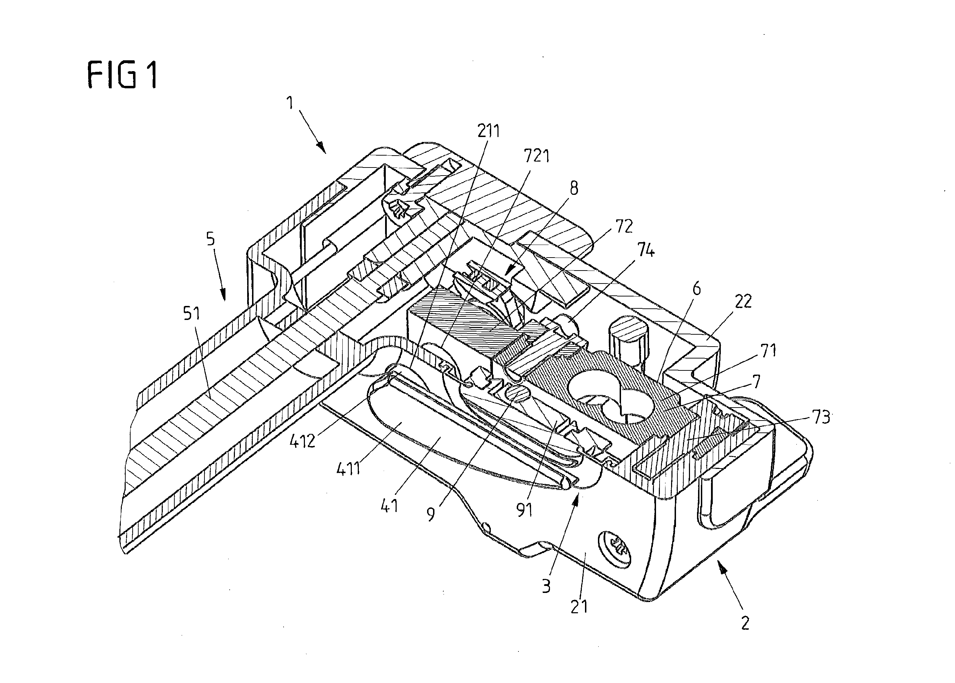

The syringe pump drive head 1 according to the invention illustrated in Figure 1 comprises a housing 2 consisting of a front part 21 that at least partially faces towards a syringe (not shown) when the drive head is in operation and a backward part 22.

Arranged in a front sidewall of the front housing part 21 is a contact portion 3 of the drive head. The contact portion 3 is configured to be pushed against the flange of a plunger of the syringe and to move the plunger relative to a barrel of the syringe in order to eject liquid from the syringe. During operation of the syringe pump the drive head is moved via a moving mechanism 5 comprising a screw drive mechanism generating a linear movement of the contact portion 3 along an axis 51 of the screw drive mechanism.

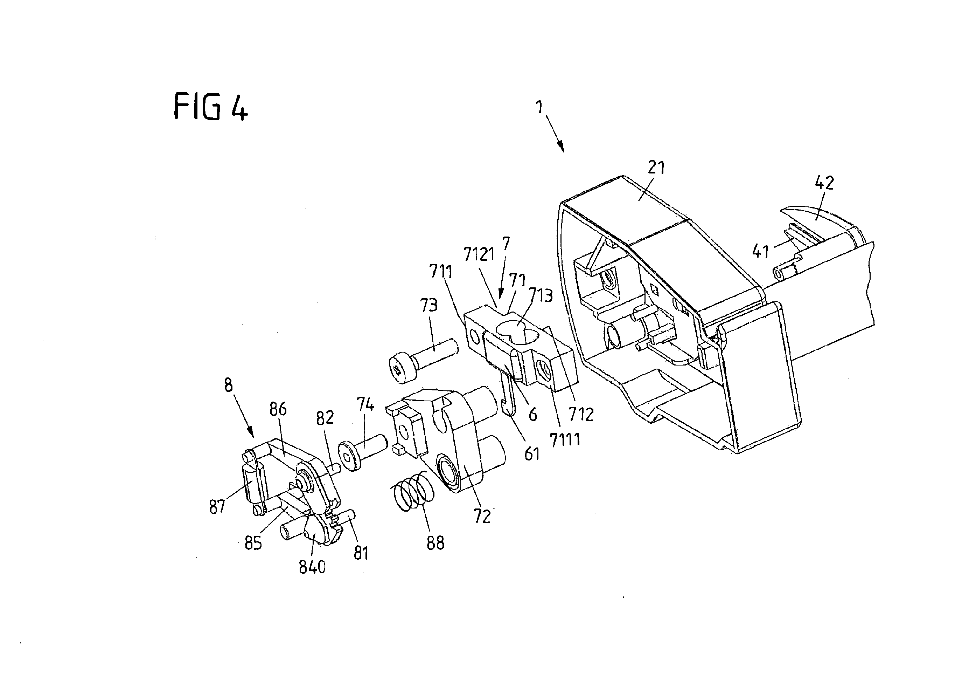

The drive head 1 further comprises two holding elements in form of rotatable anti-siphon arms 41, 42, wherein Figure 1 shows a part of one of the arms, only. Both arms are shown for example in Figure 4.

The arms 41, 42 are configured to engage a flange of the syringe plunger in order to prevent a siphon movement of the plunger, i.e. a movement away from the contact portion 3. For this, the arms 41, 42 each comprise a gripping element 411, 421 and a shaft 412, 422 connected to the gripping element 411, 421 such that the gripping elements 411, 421 can be rotated via the shaft 412, 422 between an open position in which the arms (i.e. the gripping elements) do not engage the flange of the syringe plunger and a closed position in which the arms engages the flange and, for example, hold the plunger flange in contact with the contact portion 3 of the drive head.

The drive head 1 further comprises a pressure sensor in form of a strain gauge 6 (not visible in Figure 1) for determining an inner pressure in the syringe. The strain gauge 6 is attached to a pressure sensor support 7 arranged within the housing 2. The pressure sensor support 7, in turn, is arranged and configured in such a manner that a force exerted by the syringe plunger on the contact portion 3 is transferred via the pressure sensor support 7 to the strain gauge 6. That is, the force applied to the bearing section 3, and thus the inner pressure in the syringe, can be measured using an electrical signal generated by the strain gauge 6. Although a strain gauge is used as pressure sensor in this embodiment, other suitable pressure sensors could also be used.

The shafts 412, 422 of the anti-siphon arms 41, 42 are mounted via the pressure sensor support 7. In particular, the pressure sensor support 7 comprises a first part 71 to which the strain gauge 6 is attached and a second part in the form of an arm mount 72 via which the shafts 412, 422 are mounted. The first part 71 is connected to the arm mount 72 via a screw 75.

The shafts 412, 422 reach through openings 211, 212 in the front housing part 21 and through openings 721, 722 formed by the arm mount 72. Further, the shafts 412, 422 are connected to means for generating a rotational movement of the arms 41, 42 as will be explained in more detail further below.

Further more, the shafts 412, 422 are solely mounted via the arm mount 72, i.e. they reach through openings 211, 212 of the front housing part 21, but they do not lie against the inner edge of the openings 211, 212 during the operation of the drive head ("floatingly mounted" arms). Thus, it is avoided that movements of the arms 41, 42 are transferred to the contact portion towards the strain gauge 6 during drive head operation such that perturbations of the pressure sensor signal due to such arm movements are reduced or completely avoided.

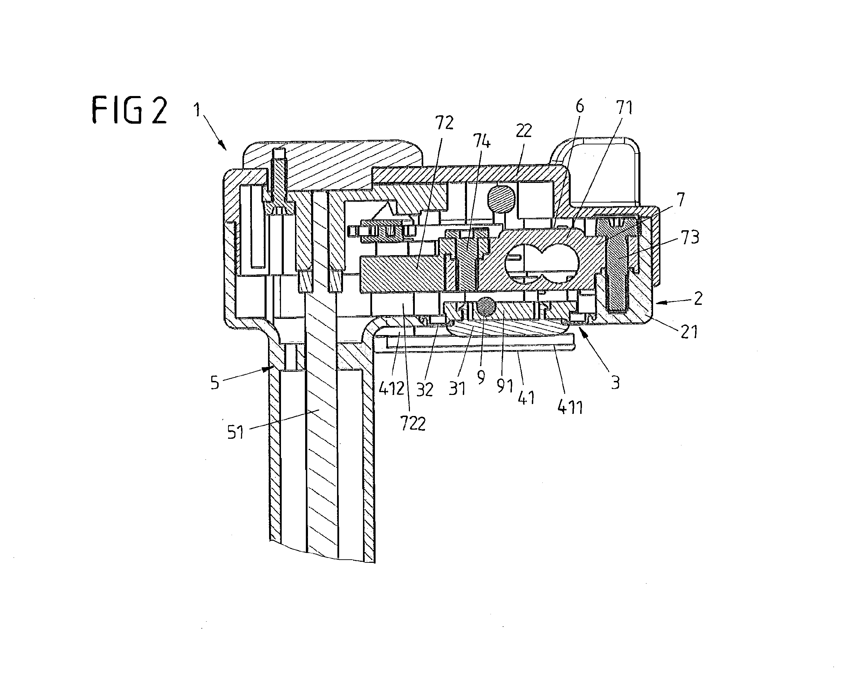

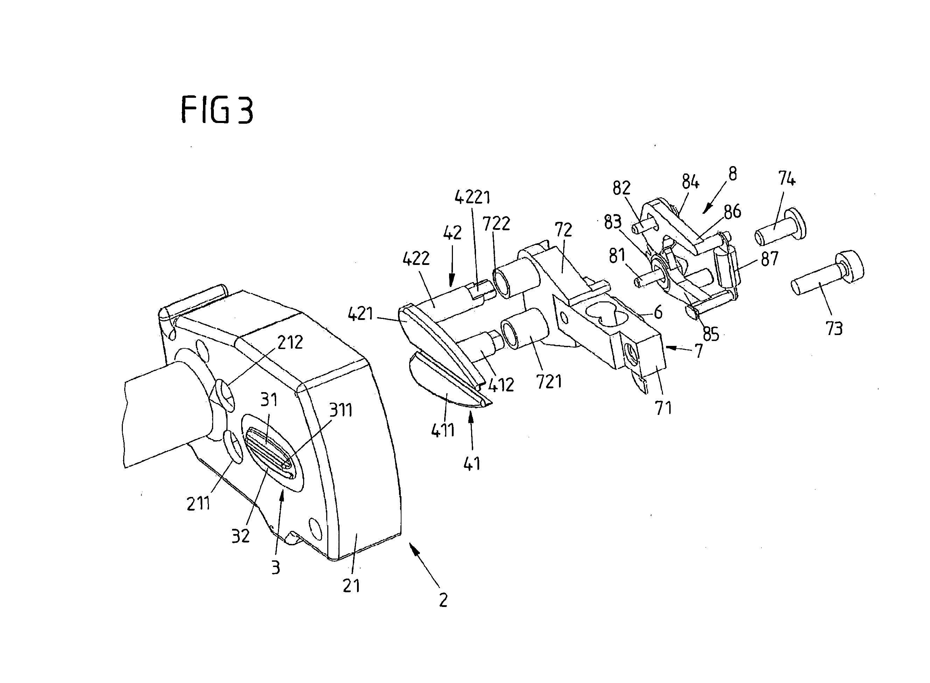

As best shown in Figures 2 and 3, the contact portion 3 comprises a contact element 31 to which the end face of the syringe plunger flange will bear when the drive head is in operation. The contact element 31 comprises (as shown in Figure 3) an oval basic section and a longitudinal protrusion 311 protruding from the basic section (and thus, from the front housing part 21) which will be in mechanical contact with the plunger flange end face.

The contact element 31 is mounted to the front part 21 of the housing 2 via a flexible (and elastic) element in form of a ring-shaped membrane 32. The membrane 32 on the one hand is connected (e.g. adhesively bonded) to the contact element 31 and on the other hand to the front housing part 21, wherein the connection of the membrane 32 to the contact element 31 and the housing part 21 may be a sealed connection. Also, the material of the membrane and its thickness may be chosen in such a way that a movement of the contact portion 31 will be transferred towards the pressure sensor support 7 and thus towards the strain gauge 6 as accurate as possible.

As also shown in Figure 2, the pressure sensor support 7 is connected to the housing 2 (i.e. the front housing part 21) via a single connection point, only, wherein the connection point is realized by a screw 73. It is noted that instead of a screw other means for realizing the single connection point are conceivable, for example, a latching connection or adhesive bonding. By providing a single connection point only, the housing and the pressure sensor are separated as much as possible in order to avoid that deformations of the housing are transmitted to the pressure sensor.

Further more, the drive head 1 comprises a pressure transmitting element in form of a spherical element 9, which is arranged in a carrier plate 91. The carrier plate 91 and thus the spherical element 9 will be moved when the syringe plunger presses against the contact portion 3 such that a section of the spherical element 9 will be pushed against the first part 71 of the sensor support 7. Therefore, a force exerted on the contact portion 3 will be transferred via the plate 91, the spherical elements 9 and the support part 71 towards the strain gauge 6. It is possible that the carrier plate 91 is pivotably mounted to the housing 2 such that if the bearing section 3 is moved (deflected) the carrier plate 91 will perform a pivot movement towards the pressure sensor support 7, thereby pushing the spherical element 9 against the pressure sensor support 7.

Figure 3 shows an exploded and perspective view of some of the components of the drive head 1 of Figures 1 and 2. Although the arms 41, 42 are shown on an interior side of the front housing part 21 in Fig. 3, it is obvious that in the assembled state of the drive head, the gripping elements 411, 421 will be located outside the front housing part 21 and the shafts 412, 421 will extend through the openings 211, 212 of the housing part 21.

The shafts 412, 422 of the arms 41, 42 are mounted in openings 721, 722 of the arm mount 72 of the pressure sensor support 7. A portion of the openings 721, 722 is formed by hollow cylindrical parts protruding from a basic portion of the arm mount 72, the shafts 412, 422 reaching through the hollow cylindrical parts towards means 8 for generating a rotational and axial movement of the arms 41, 42. The means 8 comprise axles 81, 82 that are connected to the shafts 412, 422 and may also partially reach through the arm mount 72.

Further, the axles 81, 82 are coupled to gear wheel segments 83, 84 such that a rotatable connection is established between the axles 81, 82. Further, connecting arms 85, 86 extend from the axles 81, 82, wherein the free ends of the connecting arms 85, 86 are connected to a spring 87 biasing the connecting arms 85, 86 in such a way that the anti-siphon arms 41, 42 tend to remain in their closed position. The principle of the means 8 will be explained in more detail with respect to Figures 4 to 6.

Figure 4 shows the components illustrated in Figure 3 in another perspective, wherein the strain gauge 6 can be seen to be connected to a backward surface 711 of the first part 71 of the sensor support 7. Further shown is a cable 61 connecting the strain gauge 6, for example, to a voltage supply and/or means for evaluating a sensor signal (not shown). The backward surface 711 runs parallel to a front surface 712 of the first support part 71. Both the backward and the front surface 711, 712 comprise a notch 7111, 7121 to provide a connecting portion through which the screws 73 and 74 extend in order to connect the sensor support 7 to the housing 2 and to connect the arm mount 72 to the first sensor support part 71, respectively.

Further more, the first support part 71 comprises a through opening 713 running essentially parallel to the backward and the front surface 711, 712. The through opening 713 has a cross section (along a plane that extends perpendicular with respect to the backward and the front surface 711, 712) that is composed of two overlapping circles, wherein the circle centres are positioned along a line running parallel to the backward and the front surface 711, 712. It is due to this design of the through opening 713 that if the front surface 712 is deformed under a load applied via the bearing section 3, the backward surface 711 deforms in a similar way such that the backward and the front surface 711, 712 still run parallel to one another even is the support part 71 is deformed.

In particular, the sections of backward and the front surface 711, 712 located on a side of the through opening 713 that faces towards the arm mount 72 and on a side that faces away from the arm mount 72, respectively, will still run parallel to one another and parallel to the plane in which the backward and the front surface 711, 712 originally extended (before a deformation of the support part 71) even if a force is applied to the front surface 711. This has the effect that the arm mount 72, which is connected to a section of the support part 71 located laterally to the through opening 713, will not be inclined if the sensor support part 71 deforms. Rather, it might only be displaced along the direction of movement of the drive head such that the arms 41, 42 may only be shifted along the syringe axis but will not (or at least only slightly) incline with respect to the syringe plunger flange.

Moreover, the means 8 for generating a rotational and axial movement not only generate a rotational movement of the arms 41, 42 but also a linear movement in order to be able to move the arms a certain distant away from the front housing part 21 when the syringe is arranged on the drive head. As shown in Figure 4 another spring 88 is provided between the arm mount 72 and the lower connecting arm 85, the spring 88 tending to hold thearms in a backward position. Thus, the spring 88 will exert a reset force on the lower connecting arm 85 (and as this arm is connected to the upper connecting arm 86 also on the upper connecting arm) such that the anti-siphon arms 41, 42 will tend to return towards their backward position where they hold the syringe plunger in contact with the contact portion 3.

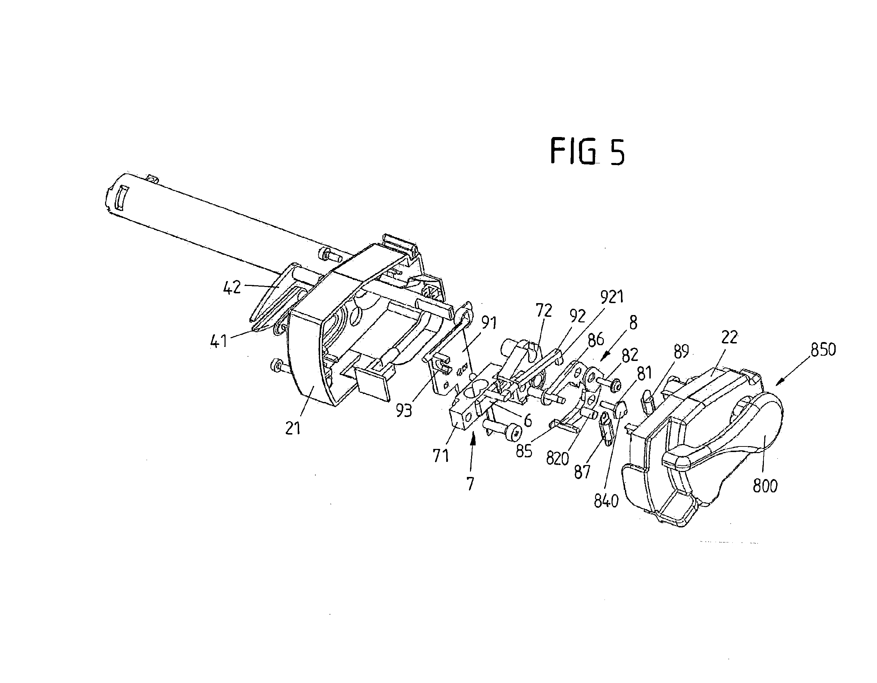

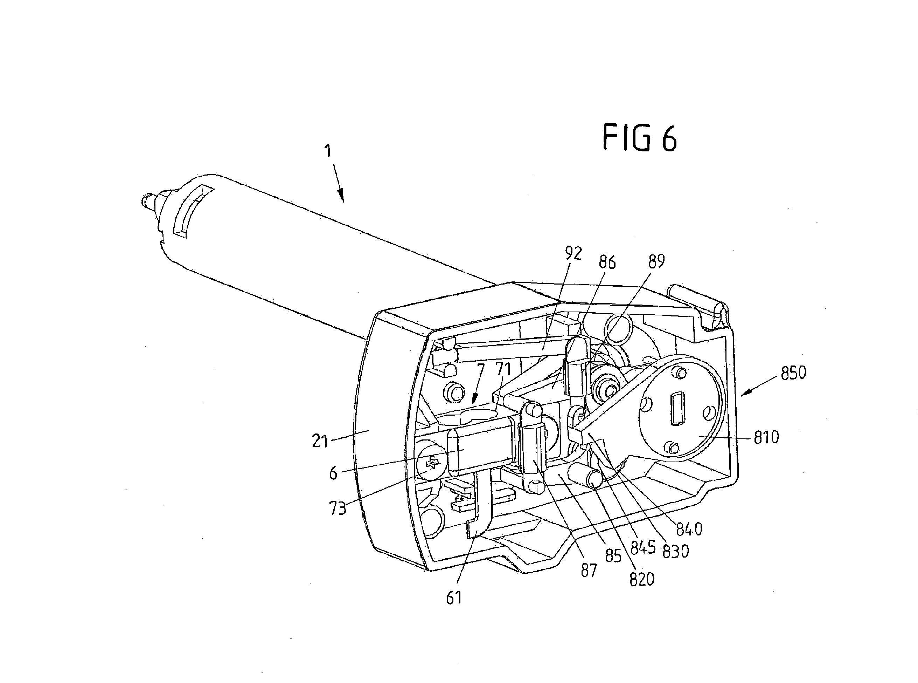

Figures 5 and 6 illustrate the components of the means 8 for generating a rotational and axial movement of the anti-siphon arms 41, 42 in more detail.

The means 8 comprise an actuating device 850 including a lever 800 that is arranged outside the housing 2. The lever 800 is connected through a side wall of the housing to a transmission component 810 located in the interior of the housing 2. Thus, a rotation of the lever 800 will cause the transmission component 810 to rotate, too (i.e. the transmission component 810 will follow the rotation of the lever).

Further more, the means 8 comprise a first interacting element in the form of a projection 830 of the transmission component 810 (i.e. the projection 830 is integrally connected to the actuating device 850) and a second interacting element in the form of a cylindrical protrusion 820 connected to the lower connecting arm 85 (thus the protrusion 820 is indirectly connected to the anti-siphon arms 41, 42 via the connection between the connecting arms 85, 86 and the arms 41, 42).

If the lever 800 is rotated from a starting position in anti-clockwise direction the projection 830 will be pushed against the protrusion 820 such that the lower connecting arm 85 and the upper connecting arm 86 will be rotated against the biasing force of spring 87. Due to the rotation of the connecting arm the anti-siphon arms 41, 42 will be rotated also such that by rotating the lever 800 in anti-clockwise direction the arms 41, 42 can be rotated from a closed position into an open position, in which the distance between the arms is larger than in the closed position.

The actuating mechanism 850 further comprises a further (third) interacting element in the form of a tapered end 840 of the lower axis 81. Assigned to the tapered end 840 is a fourth interacting element in the form of an interacting portion 845 of the transmission component 810, wherein the interacting portion 845 will push against the tapered end 840 if the lever is pivoted in anti-clockwise direction such that the lower axis 81 and the connected upper axis 82 will be moved axially (along the direction of movement of the drive head) from a backward position towards a forward position.

Following the linear movement of the axles 81, 82 the anti-siphon arms 41, 42 will move axially also, i.e. away from the contact portion 3 into the forward position. Thus, by rotating the lever 800 the anti-siphon arms 41, 42 can be moved radially and axially from a first position (closed arms, backward position with respect to the contact portion 3) into a second position (open arms, forward position with respect to the contact portion 3). A "forward position" of the arms means that their distance from the contact portion 3 (i.e. from the housing 2) is larger than in the "backward position".

The drive head 1 further comprises a latching mechanism that locks the arms 41, 42 after they have been moved in the open and forward position by rotating the lever 800. The latching mechanism comprises an engagement element 92, wherein after a rotation of the lever 800 in the open and forward position a projection 921 of the engagement element 92 engages a groove 4221 (Figure 3) provided in the shaft 422 of the upper arm 42 in such a way that the arms 41, 42 are locked in the open and forward position. Also, as the lever 800 is biased via spring 89, it will return in its starting position if the arms 41, 42 are locked in the open position. In the starting position of the lever 800 the projection 830 and the interacting portion 845 are not any more in contact with the protrusion 820 and the tapered ending 840, respectively, such that the arms 41, 42 are decoupled from the transmission component 810 and the lever 800.

The engagement element 92 is held in a section 93 of the plate carrier 91, wherein carrier plate will be moved awy from the housing 2 if the syringe plunger pushes against the contact element 31. Thus the engagement element is moved also and released from its locked position such that the arms 41, 42 are not locked anymore. If the syringe is removed from the drive head, the contact element will return to its outward position again due to the elasticity of the surrounding membrane 32. A spring may be used instead or in addition to a membrane.

It is noted, however, that the described latching mechanism is only optional. It is also possible that the arms 41, 42 are held in the open position manually, i.e. by holding the lever 800 in its "open" position during the arrangement of the syringe at the drive head. After the syringe is arranged the lever 800 is releases such that the arms 41, 42 snap back into the closed and backward position.

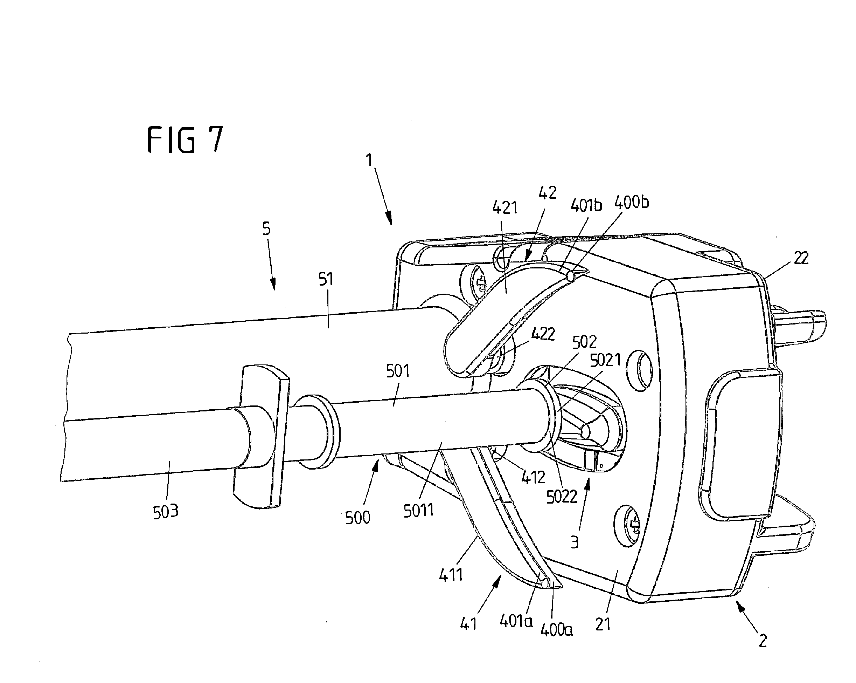

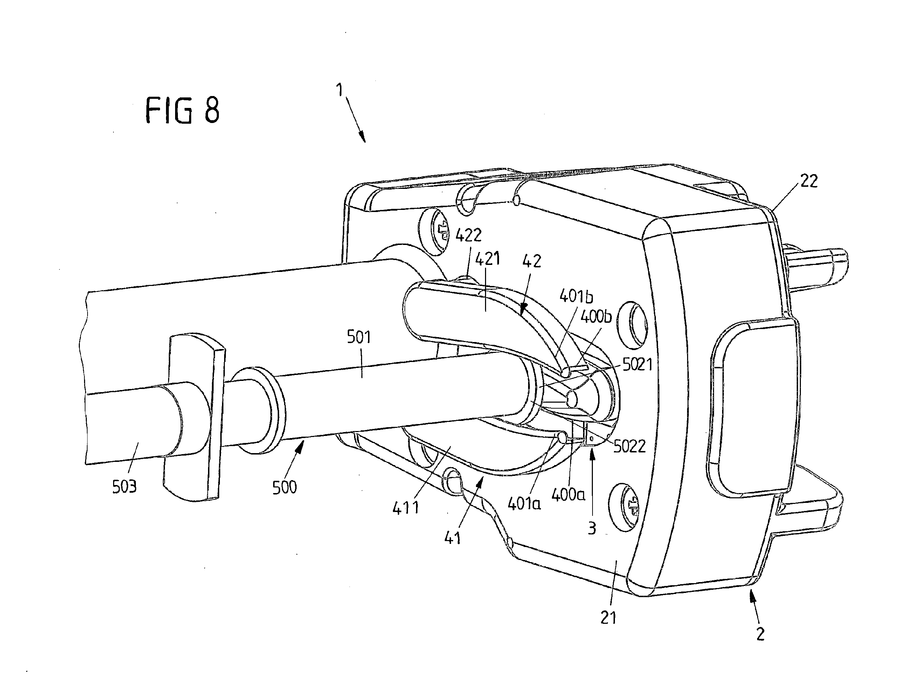

Figures 7 and 8 depict further views of the drive head 1, wherein a syringe 500 is shown to be arranged at the drive head, the syringe 500 comprising a plunger 501. A typical syringe plunger as shown in Figure 7 comprises a main portion 5011 and a flange 502 arranged at an end of the main portion, the flange protruding from the main portion, i.e. the flange 502 has a larger diameter than the plunger main portion 5011.

The syringe is arranged at the drive head in such a way that an end face of the flange 502 of the syringe plunger 501 bears against the contact portion 3 of the drive head such that when the contact portion 3 is moved, the plunger 501 will be moved within a barrel 503 of the syringe 500 such that liquid will be ejected from the syringe.

As set forth above, the drive head comprises means 8 for generating a rotational and axial movement of the anti-siphon arms 41, 42 in order to move the arms from an open and forward position ("second position", Fig. 7) into a closed and backward position ("first position", Fig. 8). After the arrangement of the syringe at the drive head the arms 41, 42 will snap back into the first position, i.e. into the closed and backward position, in such a way that the gripping elements 411, 421 bear against a peripheral edge 5021 of the flange 502.

More over, the gripping elements 411, 421 each comprise a first section 400a, 400b and a second section 401 a, 401 b protruding beyond the first section, wherein in the closed and backward position of the arms 41, 42 only the first sections 400a, 400b of the gripping elements 411, 421 bear against the peripheral edge 5021 of the flange 502, whereas the second sections 401 a, 401 b do not bear against the peripheral edge 5021 but lie against a front surface (front side) 5022 of the flange 502 that faces away from the contact portion 3.

It is noted, however, that it is also possible that the second sections 401 a, 401 b do not lie against the flange front surface 5022 in the closed and backward position of the arms 41, 42. In that case the second sections 401 a, 401 b are positioned in a distance from the front surface 5022 but are still positioned in such a way that they can block a siphon movement of the arms 41, 42, i.e. they still "engage" the plunger flange.

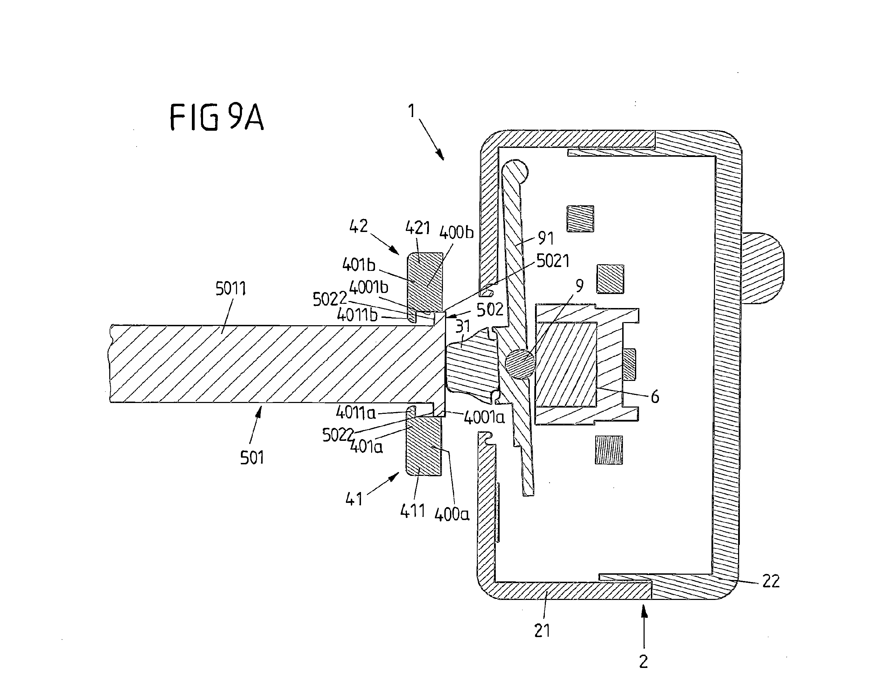

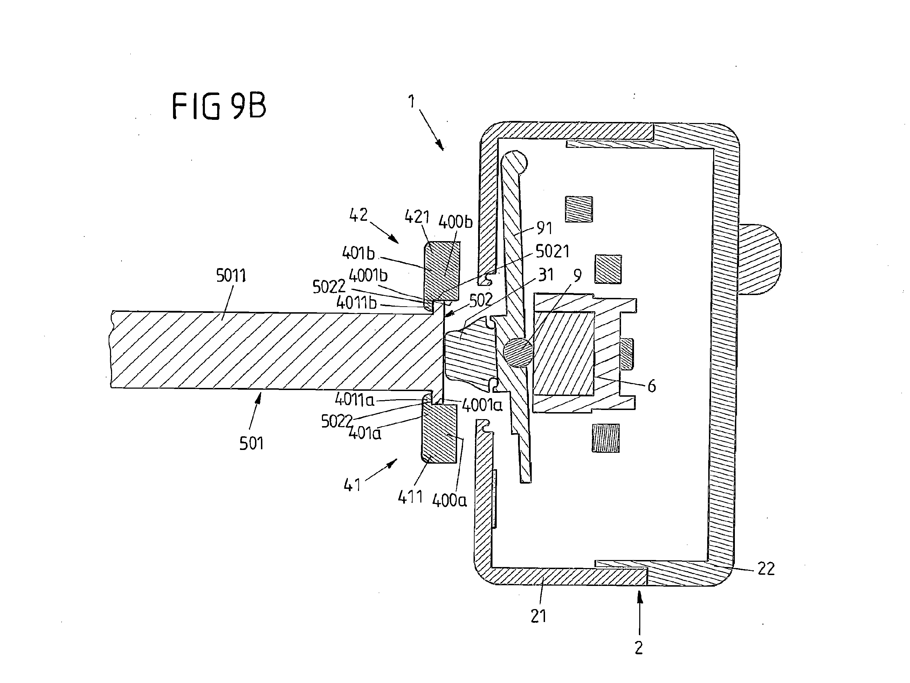

Figures 9A and 9B show sectional views of the drive head 1, wherein according to Fig. 9A the arms 41, 42 are in their closed positions but do not have yet reached the most backward position. After the arms 41, 42 were rotated by the means 8 (i.e. via the return force of the springs 87-89) until they bear against the peripheral edge 5021 of the flange 502 (as shown in Fig. 9A), the backward movement of the arms starts or continues (also by means of the return force of the springs 87-89) until the arms 41, 42 bear against the inner surface surface 5022 of the flange 502 as shown in Figure 9B.

In that example, the rotational movement and the axial movement are at least partially carried out successively. In order to generate this sequential movement the spring 87 (generating the rotational movement of the arms towards the closed position) and the spring 88 (generating the linear movement of the arms towards the backward position) are adapted accordingly. For example, the spring 87 comprises a higher spring constant than spring 88.

It is noted, however, that it is also possible that the rotational and the axial movement of the arms (from the second into the first position) is carried out simultaneously (or at least essentially simultaneously).

It is further noted that in the forward position the distance of the arms 41, 42 from the contact portion 3 is smaller than the thickness of the plunger flange 502 such that even if the arms are rotated in the closed position before being moved in the backward position it is avoided that the arms pass the flange 502. Thus, it is ensured that the arms in the closed position will bear against the peripheral edge 5021 of the flange 502.

The second sections 401 a, 401 b of the gripping elements 411, 421 extend beyond the first sections 400a, 400b such that "fingers" 4011a, 4011 b protrude beyond an inner surface 4001 a, 4001 b of the first sections 400a, 400b. The length of the fingers 4011a, 4011b is smaller than the height of the plunger flange 502 such that inner surfaces 4001 a, 4001 b of the first sections 400a, 400b of the gripping elements bear against the peripheral edge surface 5021 of the flange 502 while the second sections 401 a, 401 b are not in contact with a main portion 5011 of the plunger.

As the fingers 4011a, 4011b are arranged perpendicular to the inner surfaces 4001 a, 4001b of the first sections 400a, 400b an edge is formed between the first and the second sections, wherein the edge - viewed perpendicular to the plunger axis - extends essentially along the whole length of the gripping elements 411, 421. Further, the fingers 4011a, 4011b are slightly tapered towards the plunger 501, the tapering being realized by a bending of a front side portion of the fingers 4011a, 4011b.

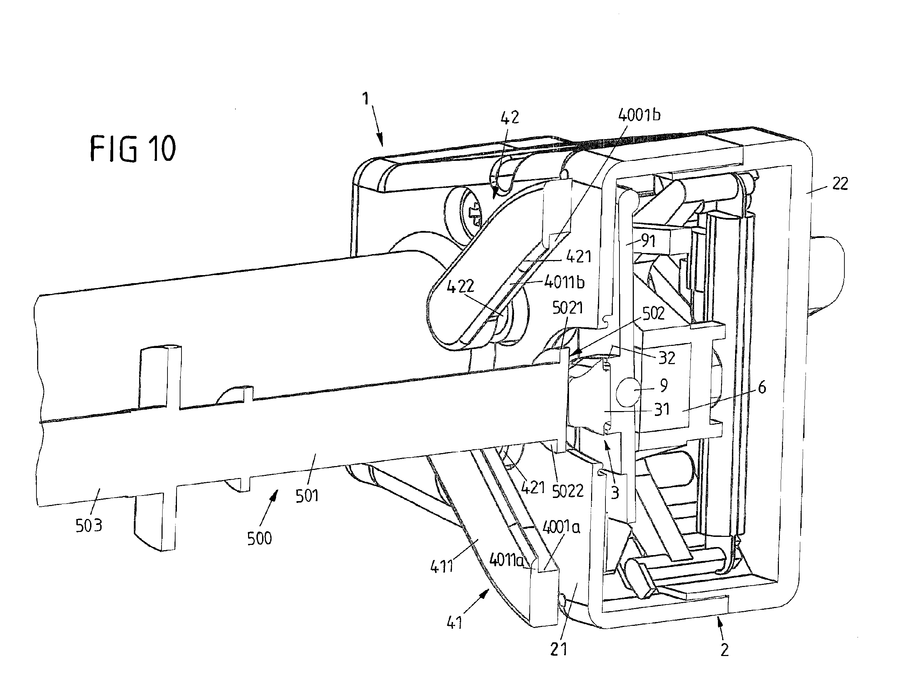

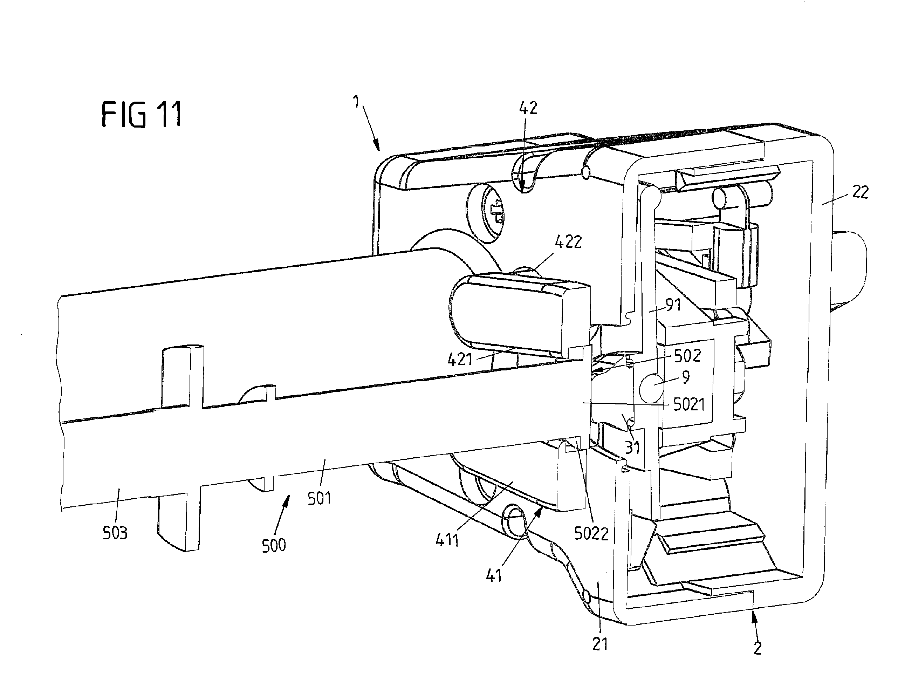

Figures 10 (related to the open position of the arms 41, 42) and 11 (closed position) provide perspective views corresponding to Figures 7 and 8, respectively, wherein according to Fig. 11 the arms are in their closed but not yet in their most rearward position. It can be seen that the carrier plate 91 arranged between the finger 31 of the bearing portion 3 and the pressure sensor 6 may be mounted to a hinge portion of the housing such that a force applied to the finger 31 causes the support plate 91 to rotate such that the spherical element 9 is pushed against the pressure sensor device 6 by a rotational movement of the support plate 91.

It is noted that the principle of the means 8 described with respect to Fig. 7 to 9 (moving the arms so as to bear against a peripheral edge of the flange in the first position) can be used with any kind of a syringe pump drive head. In particular, the principle of letting the arms 41, 42 bear against a peripheral edge of the flange can be used independently from the concept of floatingly mounting the arms 41, 42 as described with respect to Fig. 1 to 6, i.e. the principle of letting the arms 41, 42 bear against a peripheral edge of the flange can be used also in connection with holding elements that are conventionally mounted, e.g. via the drive head housing.

### Reference signs

- 1: drive head
- 2: housing
- 3: contact portion
- 5: moving mechanism
- 6: strain gauge
- 7: pressure sensor support
- 8: means for generating rotational and axial movement
- 9: spherical element
- 21: front part
- 22: back part
- 31: contact portion
- 32: membrane
- 41: first arm
- 42: second arm
- 51: axis
- 61: cable
- 71: first part
- 72: arm mount
- 73,74: screw
- 81,82: axis
- 83, 84: gear wheel segment
- 85, 86: arm
- 87, 88, 89: spring
- 91: carrier plate
- 92: engagement element
- 93: latching structure
- 211,212: through opening
- 311: protrusion
- 400a, 400b: first section
- 401 a, 401 b: second section
- 411, 421: gripping element
- 412,422: shaft
- 500: syringe
- 501: plunger
- 502: flange
- 503: barrel
- 711: backward surface
- 712: front surface
- 713: through opening
- 721,722: opening
- 800: lever
- 810: transmission component
- 820: protrusion
- 830: projection
- 840: tapered end
- 845: interacting portion
- 850: actuating device
- 4001a, 4001b: inner surface
- 4011a,4011b: finger
- 4221: groove
- 5011: main portion
- 5021: peripheral edge
- 5022: front surface
- 7111, 7121: notch

## Claims

1. A drive head for a syringe pump, comprising:
- a contact portion (3) configured to bear against a plunger (501) of a syringe (500) in order to move the plunger (501) relative to a barrel (503) of the syringe, wherein the plunger (501) comprises a main portion (5011) and a flange (502) protruding from the main portion;
- at least one holding element (41, 42) for holding the flange (502) of the syringe plunger (501) in order to prevent a siphon movement of the plunger (501) away from the contact portion (3);
- means (8) for generating a rotational and/or a linear movement of the holding element (41, 42) in order to move the holding element (41, 42) from a first position into a second position and vice versa, wherein in the first position the holding element (41, 42) holds the flange (502) of the syringe plunger (501) while in the second position the holding element (41, 42) is positioned with a distance to the flange (502),
**characterized in that**
the means (8) are configured in such a way that after arranging a syringe at the drive head such that the plunger (501) bears against the contact portion (3) the holding element (41, 42) will bear against a peripheral edge (5021) of the plunger flange (502) when being moved into the first position.

2. The drive head according to one of the preceding claims, **characterized in that** the means (8) comprise resilient means for pre-tensioning the holding element (41, 42) such it tends to return into the first position.

3. The drive head according to claim 1 or 2, **characterized in that** in the second position the holding element (41, 42) is positioned with a greater distance from the contact portion (3) than in the first position.

4. The drive head according to claim 3, **characterized in that** in the second position the holding element (41, 42) is positioned with a distance to the contact portion (3) that is smaller than the thickness of the plunger flange (502) such that the holding element (41, 42) will not pass the peripheral edge (5021) of the plunger flange (502) when moving from the second into the first position.

5. The drive head according to one of the preceding claims, **characterized in that** the holding element (41, 42) comprises a gripping element (411, 421) having a first section (400a, 400b) that in the first position of the holding element bears against the peripheral edge (5021) of the plunger flange (502) and a second section (401 a, 401 b) that in the first position of the holding element is located with a distance from the main portion (5011) of the plunger (502) while engaging the flange (502) on a side that faces away from the contact portion (3).

6. The drive head according to claim 5, **characterized in that** the second section (401 a, 401 b) protrudes beyond the first section (400a, 400b) such that a step is formed between the first and the second section.

7. The drive head according to claim 5 or 6, **characterized in that** gripping element (411, 421) exclusively bears against the plunger flange (502) via the first section (400a, 400b).

8. The drive head according to one of the claims 5 to 7, **characterized in that** the holding element (41, 42) comprises a rotatably mounted shaft (412, 421) connected to the gripping element (411, 421) so as to rotate the gripping element (411, 421) from an open position in which it does not bears against the plunger flange (502) into a closed position in which it bears against the plunger flange (502), and vice versa.

9. The drive head according to one of the preceding claims, **characterized by** a first and a second holding element (41, 42) that each can be moved from a first position into a second position by the means (8), wherein in the first position both the first and the second holding element bear against the peripheral edge (5021) of the plunger flange (502) such that the syringe plunger is centered between the first and the second holding element (41, 42).

10. A syringe pump comprising a drive head according to one of the preceding claims.

11. Method for operating a drive head of a syringe pump, comprising the steps of:
- providing a drive head (1) for a syringe pump, in particular according to one of the preceding claims;
- arranging a syringe (500) on the drive head (1), the syringe comprising a plunger (501) having a main portion (5011) and a flange (502) protruding from the main portion (5011), wherein the drive head (1) comprises a contact portion (3) configured to bear against the plunger (501) of the syringe (500) in order to move the plunger (501) relative to a barrel (503) of the syringe, and at least one holding element (41, 42) for holding the flange (502) of the syringe plunger (501) in order to prevent a siphon movement of the plunger (501) away from the contact portion (3),
**characterized by**
moving the holding element (41, 42) into a position in which it bears against the peripheral edge (5021) of the plunger flange (502).

12. The method according to claim 11, **characterized in that** the holding element (41, 42) is moved from a first position into a second position prior to the arrangement of the syringe at the drive head and is returned into the first position after the arrangement of the syringe, wherein in the second position the holding element (41, 42) is positioned with a distance to the flange (502) and in the first position the holding element (41, 42) bears against the peripheral edge (5021) of the plunger flange (502).

13. The method according to claim 11, **characterized in that** the holding element (41, 42) is moved from the first into the second position against the return force of resilient means and returned into the first position by means of the return force of the resilient means.

14. The method according to one of the claims 11 to 13, **characterized in that** the holding element (41, 42) comprises a gripping element (411, 421) that has a first section (400a, 400b) and a second section (401 a, 401 b), the second section (401 a, 401 b) protruding beyond the first section (400a, 400b), wherein after arranging the syringe at the drive head the holding element (41, 42) is moved into a position in which the first section bears against the peripheral edge (5021) of the plunger flange (502) while the second section is located in a distance to main portion (5011) of the plunger (501).

15. Method according to claim 14, **characterized in that** the holding element (41, 42) is moved into a position in which the first section (400a, 400b) bears against the peripheral edge (5021) of the plunger flange (502) while the second section (401 a, 401 b) is located in a distance to the main portion (5011) of the plunger (501) and bears against a surface of the flange (502) that faces away from the contact portion (3).
